(19) Europäisches Patentamt
European Patent Office
Office européen des brevets

(11) **EP 1 825 807 A2**

(12) **EUROPEAN PATENT APPLICATION**

(43) Date of publication:
**29.08.2007 Bulletin 2007/35**

(51) Int Cl.:
*A61B 5/053* (2006.01)    *G01N 27/02* (2006.01)
*G01N 33/487* (2006.01)

(21) Application number: **06026522.0**

(22) Date of filing: **21.12.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **28.02.2006 US 363922**

(71) Applicant: **St. Jude Medical AB
175 84 Järfälla (SE)**

(72) Inventors:
• **Öhlander, Malin
118 48 Stockholm (SE)**
• **Blomqvist, Andreas
163 43 Spånga (SE)**
• **Holmström, Nils
175 57 Järfälla (SE)**

(54) **Implantable medical device with impedance measuring circuit**

(57) The present invention relates to a method for detecting a change of a condition of a patient using an implantable medical device (10; 20; 20') being connectable to the patient in at least one electrode configuration. The method comprises the steps of: initiating (54) at least one impedance measurement session to obtain at least one impedance value corresponding to an impedance of whole blood of the patient, calculating (60) at least one relative or absolute value of an amount of hematocrit in the blood of the patient using the at least one impedance value; and determining (62, 64) a present hematocrit level by means of the at least one hematocrit value, wherein a change of the condition can be derived from the present hematocrit level of the patient. Furthermore, the invention relates to an implantable medical device (10; 20; 20') and a computer readable medium comprising instructions for bringing a computer to perform the method.

Fig. 4

EP 1 825 807 A2

**Description**

TECHNICAL FIELD

The present invention generally relates to implantable medical devices, such as cardiac pacemakers and implantable cardioverter/defibrillators, and in particular to a method and medical device for detecting or monitoring a change of a condition of a patient.

BACKGROUND OF THE INVENTION

Hematocrit is a common parameter used by physicians to diagnose and monitor different medical conditions of a patient, such as, CHF, cancer, chronic kidney disease, diabetes, rheumatoid arthritis, inflammatory bowel disease and HIV/AIDS. A trend over the changes on the hematocrit value of a patient would therefore be a valuable parameter for monitoring a patient suffering from such a condition. The hematocrit indicates the proportion of cells and fluids in the blood and the hematocrit is, in practice, the percent of whole blood that is composed of red blood cells (Erythrocytes). In men 39-55 % of the blood volume is made up of red blood cells and in women 36-48 %. A low hematocrit value may be the result from either an increased plasma volume (hemodilution) or from a reduced red blood cell volume (true anemi). In patients suffering from CHF, low hematocrit values has been found to be of a frequent occurrence and is, also, associated with a poor prognosis, see, for example, "Hemodilution is Common in Patients with Advanced Heart Failure", Androne et al., Circulation. 2003;107:226-229.

In "The Cardio Renal Anemia (CRA) Syndrome: Congestive Heart Failure, Cronic Kidney Insufficiency, and Anemia", Silverberg et al., Dialysis Times, News & Views from RPI, Volume 10, No. 1, it is shown that the presence of anemia is associated with a more severe degree of CHF and with an increased mortality in CHF. In addition, it is shown that there exits a strong correlation between the severity of anemia and hospitalization and/or length of stay. The anemia caused by the CHF is not merely hemodilutional, but also due to a reduction in red cell volume, which, in turn, may be due to primarily two factors: the renal damage caused by the CHF causes a reduced production of EPO (erythropoietin) in the kidneys and CHF itself may cause anemia. Animal studies have confirmed that anemia is common in CHF.

In inflammatory bowel disease anemia is an indicator of disease severity and in HIV/AIDS patients anemia has a serious impact on the quality of life of the patients and is also strongly associated with disease progression and in and increased risk of death.

Furthermore, patients suffering from CHF are often afflicted by cardogenic pulmonary edema, which is caused by the accumulation of fluid in the lung insterstitium and alveoli due to the fact that the left ventricular venous return exceeds left ventricular cardiac output. That is, more fluids are transported to the lung region than from the lung region causing the accumulation of fluids. Incipient pulmonary edema is hence an effective indicator of CHF and, due to the fact that CHF often leads to low hematocrit values as discussed above, a low hematocrit value may be an early indicator of an accumulation in pulmonary edema.

Accordingly, there is great need of efficient methods for measuring or estimating the hematocrit values of patients in order to detect or monitor different conditions of the patients, such as, CHF, cancer, chronic kidney disease, diabetes, rheumatoid arthritis, inflammatory bowel disease and HIV/AIDS. In light of this, several studies have been performed on estimating the hematocrit value or count of patients. In particular, impedance or resistivity measurements of the blood of a patient have been found to constitute an effective parameter when estimating the hematocrit value or count. For example, in "An electronic method for rapid measurement of hematocrit in blood samples" by Cha K. et. al. Physiol. Meas. 15 5 (1994) 129-137, the resistance and reactance of a constant volume of blood were measured at a low (50 kHz) and a high (1 MHz) frequency, and these dual-frequency impedance measurements were used to determine the intracellular and extracellular (plasma) fluid volume and the hematocrit in blood samples.

In "The changes in blood resistivity with hematocrit and temperature", by Mohapatra S.N., et. al. it is shown that the resitivity of whole blood increases with an increase in hematocrit and a decrease in temperature. Thus, it is recognized that the electrical impedance of blood varies with hematocrit and there is a well-known correlation between the impedance of whole blood and the ratio between plasma and red blood cells (or in fact blood cells in general). Higher impedance indicates a larger amount of blood cells (or lower plasma volume) and vice versa (a large number of red blood cells = a high hematocrit value = a higher resistivity or impedance value). However, these studies have been performed ex. vivo, i.e. on blood samples. Conventionally, the blood sample is acquired from a vein of a patient via, for example, a syringe. The blood, contained in an elongated vessel, is then centrifuged and the height percentage of the column blood in the vessel, which is solid, represents the hematocrit.

More recently, elaborate and expensive cell counting laboratory instruments has been more common. But, as with the centrifuge method, the blood must still be invasively removed from the patient for analysis. A non-invasive method would be highly desirable because it would subject the patient to less pain and inconvenience and would preserve the patient's blood for its normal functions.

International patent application WO 99/09883 describes a system and method for in-vivo hematocrit measurement using impedance and pressure plethysmography. The hematocrit of blood perfusing a finger of a patient is determined by stimulating the finger with two current frequencies, wherein one is relatively high (e.g. 10 MHz) and one is relatively low (e.g. 100 kHz). The hematocrit is determined using the ratio of the high pulsatile component and the low frequency pulsatile component. In U.S. patent No. 5,526,808 another impedance method for measuring the hematocrit non-invasively and in-vivo is described.

However, when practicing these systems and methods for non-invasively and in-vivo measuring the hematocrit of blood using the impedance, stimulation and sensor electrodes are applied to a portion of the body that contains, for example, an artery. As the impedance measured by the sensor electrodes represent not only that of the blood within the artery, but also include those of all the intracellular and extracellular components of the body portion under test, extensive filtering and processing of the overall response are required to isolate the impedance response attributable to the blood within the artery from that of the surrounding tissue. Furthermore, different conditions of the skin, e.g. whether the skin is dry or moist, may also affect the measurements. In other words, the are a number of potential error sources that may affect the accuracy and reliability of the measurements of the hematocrit using the above-mentioned prior art methods and systems. In order to monitor or detect changes of conditions of a patient, such as, CHF, cancer, chronic kidney disease, diabetes, rheumatoid arthritis, inflammatory bowel disease and HIV/AIDS it is of interest to measure the hematocrit on a substantially continuous basis, as well as during different time points of the day, for example, when the patient is sleeping, which may be difficult using the prior art methods due to the fact that, for example, sensor electrodes have to be applied on a body portion were the measurement will be performed.

According to a study discussed in "Catheter-based impedance measurements in the right atrium for continuously monitoring hematocrit and estimating blood viscosity changes; an in vivo feasibility study in swine", Gheorghe A. Pop, et al., Biosensors and Bioelectronics 19 (2004) 1685-1693, the hematocrit level of blood was determined in vivo by electrical resistivity measurements in order to monitor the whole blood viscosity. A catheter adapted to measure the electrical resistivity was attached to the right atrium wall in swine hearts exposed following a mid-line thoracotomy of the swine. This method was used in open-heart surgery.

Hence, there is a significant need for an improved method and medical device that are capable of, in an accurate and reliable way, measuring the hematocrit of a patient in vivo using the impedance of the blood in order to detect or monitor a change of a condition of a patient, such as CHF, cancer, chronic kidney disease, diabetes, rheumatoid arthritis, inflammatory bowel disease and HIV/AIDS.

BRIEF DESCRIPTION OF THE INVENTION

Thus, an object of the present invention is to provide an improved method and medical device that are capable of, in an accurate and reliable way, measuring the hematocrit of a patient in vivo using the impedance of the blood in order to detect or monitor a change of a condition of a patient, such as CHF, cancer, chronic kidney disease, diabetes, rheumatoid arthritis, inflammatory bowel disease and HIV/AIDS.

Another object of the present invention is to provide an improved method and medical device that are capable of measuring the hematocrit in-vivo using the impedance of blood on a substantially continuous basis, as well as during different time points of the day, for example, during the night.

These and other objects are achieved according to the present invention by providing a method, a medical device, and a computer readable medium having the features defined in the independent claim. Preferable embodiments of the invention are characterised by the dependent claims.

In the context of this application, the term "impedance" refers to the impedance calculated as $U=Z*I$, where Z is the impedance, I the (applied AC) current and V the (sensed AC) voltage. The impedance differs within different tissues, i.e. the dielectric properties of different tissue differ and the dielectric properties of tissue will also differ depending on the frequency of the applied AC voltage (or AC current). The dielectric properties of cells at low frequency impedance measurement are that of a non-conducting material but at high frequencies the cell membranes starts to conduct current. Thus, when performing impedance measurements in blood at low frequencies, the alternating current will mainly pass around the red blood cells and the amount of red blood cells in path of the current will thus influence the baseline impedance level. Impedance at low frequencies, below about 100 kHz, is inversely related to the relative volume of extracellular water (plasma) and thus increases as the red cell volume increases or the plasma fraction decreases. When measuring at higher frequencies, the alternating current will pass through the blood cell membranes and the impedance will differ since the impedance reflect both extracellular water (plasma) volume and some intracellular water. This is further discussed in "An electronic method for rapid measurement of hematocrit in blood samples" by Cha K. et. al., Physiol. Meas. 15 (1994) 129-137. Hence, the DC impedance level will correlate to the ratio between the plasma and the red blood cells (or blood cells). A higher DC impedance level will indicate a larger amount of blood cells (or a lower plasma volume) and vice versa (many red blood cells = high hematocrit = higher impedance value).

According to an aspect of the present invention, there is provided a method for detecting a change of a condition of a patient using an implantable medical device being connectable to the patient in at least one electrode configuration. The method comprises the steps of: initiating at least one impedance measurement session using the electrode configuration to obtain at least one impedance value corresponding to an impedance of whole blood of the patient, wherein the electrode configuration has at least two electrodes adapted to be located within the heart of the patient, calculating at least one relative or absolute value of an amount of hematocrit in the blood of the patient using the at least one impedance value, and determining a present hematocrit level dependent on the at least one hematocrit value, wherein a change of the condition can be derived from the present hematocrit level of the patient.

According to a second aspect of the present invention, there is provided an implantable medical device for detecting a change of a condition of a patient, the device being interacting with the patient via at least one electrode configuration arranged such that impedance measurement sessions can be performed within the body of the patient. The device comprises an impedance circuit adapted to measure at least one impedance of the blood of the patient at the electrode configuration, the electrode configuration having at least two electrodes arranged such that they can be located within a heart of the patient. The impedance circuit is adapted to, upon receiving a triggering signal, initiate at least one impedance sensing session to obtain at least one impedance value corresponding to an impedance of whole blood of the patient; and a controller adapted to calculate at least one relative or absolute value of an amount of hematocrit in the blood of the patient using the at least one impedance value; and to determine a present hematocrit level dependent on the at least one hematocrit value, wherein a change of the condition of the patient can be derived from a present hematocrit level.

According to a third aspect of the present invention, there is provided a computer readable medium comprising instructions for bringing a computer to perform a method according to the first aspect.

Thus, the invention is based on the idea of measuring the hematocrit of a patient in vivo using an implantable medical device using the impedance of the blood by means of a electrode configuration comprising at least two electrodes arranged such that they can be located within a heart of the patient. A change of a condition of the patient can be derived from the hematocrit level of the patient, such as CHF, cancer, chronic kidney disease, diabetes, rheumatoid arthritis, inflammatory bowel disease and HIV/AIDS.

This solution provides several advantages over the known solutions disclosed, for example, in international patent application WO 99/09883 and the U.S. patent No. 5,526,808. One advantage is that the hematocrit of the patient can be measured on a substantially continuous basis, as well as during different time points of the day, for example, when the patient is sleeping, which obviously may be difficult using the prior art methods due to the fact that, for example, sensor electrodes have to be applied on a body portion were the measurement will be performed.

Another advantage with the present invention is that the hematocrit level can be determined with a high degree of accuracy and reliability since the impedance values is measured by an electrode configuration having at least two electrodes adapted to be located within a heart of the patient, i.e. the distance between the electrodes is sufficiently small to allow a localization within, for example, an artery/vein. Thereby, influence from surrounding tissue and influence from, for example, volume changes of the blood, i.e. noise, can be minimized. Accordingly, the present invention does not require any substantive signal processing, such as filtering, in comparison with, for example, the solution presented by U.S. patent No. 5,526,808 where the impedance is measured non-invasively and in-vivo by applying electrodes to a portion of the body that contains, for example, an artery. In this known method, the impedance measured by the sensor electrodes represents not only that of the blood within the artery, but also include those of all the intracellular and extracellular components of the body portion under test and extensive filtering and processing of the overall response in order to isolate the impedance response attributable to the blood within the artery from that of the surrounding tissue are required. Furthermore, different conditions of the skin, e.g. whether the skin is dry or moist, may also affect the measurement. In other words, the are a number of potential error sources that may affect the accuracy and reliability of the measurements of the hematocrit using the above-mentioned prior art methods and systems.

In one embodiment, the at least one impedance measurement session is repeated at predetermined intervals of time, wherein a sequence of hematocrit values over time can be obtained. The series containing the hematocrit values obtained over time is stored. Thus, a trend over the changes in hematocrit over time can be determined and monitored, which, in turn, can be used when deriving a change of the condition of the patient. In one example, the development of the hematocrit level is monitored by comparing successive hematocrit values.

According to an embodiment of the present invention, the at least one impedance measurement session is correlated with the cardiac cycle of the patient such that the session is initiated before the onset of systole. Thereby, the more reliable and accurate impedance values can be obtained, which, in turn, entails that more reliable and accurate hematocrit values can be obtained.

The trend over changes of the hematocrit level can be used to monitor a patient who has Congestive Heart Failure (CHF) since low hematocrit counts frequently occurs in CHF patients, which also is associated with a poor prognosis as discussed in Circulation, 2003, Jan 21;107(2):226-9.

In one embodiment, a first impedance measurement session using a current at a first frequency using the electrode configuration is initiated in order to obtain at least one complex impedance value corresponding to an impedance of the blood of the patient at the first frequency, and a second impedance measurement session using a current at a second frequency using the electrode configuration is initiated in order to obtain at least one complex impedance value corresponding to an impedance of the blood of the patient at the second frequency, wherein the second frequency is higher than the first frequency. These complex impedance values can be used to determine a phase shift between the first complex impedance value obtained in the first impedance measurement session and the second complex impedance value obtained in the second impedance measurement session. The phase shift can be used to calculate at least one value of the amount of hematocrit in the blood of the patient using the phase shift.

According to examples of the present invention, the at least one impedance value is calculated as a mean value of the measured impedance signal over a predetermined period of time.

In a further embodiment of the present invention, the heart rate of the patient and/or the breathing rate of the patient are sensed. The impedance measurements sessions are synchronized with a specific heartbeat in the respiration cycle of the patient and the at least one impedance value is calculated as a mean value of the measured impedance signal during the heartbeat. Alternatively, the at least one impedance value is calculated as a mean value of the measured impedance signal over a predetermined number of heartbeats. In another example, the at least one impedance value is calculated as a mean value for the heartbeat over a predetermined number of respiration cycles of the patient. In yet another alternative, the at least one impedance value is calculated as a mean value of the measured impedance signal during a number of breaths. Hence, the accuracy of the impedance values can be further increased and the accuracy of the obtained hematocrit values can thus be further enhanced.

According to a further example of the present invention, the heart rate of the patient and the breathing rate of the patient are sensed. The sensed rates are used to synchronize the impedance measurement session with a part of a specific heartbeat in the respiration cycle of the patient; and the at least one impedance value is calculated as a mean value of the measured impedance signal during the part of the heartbeat. Thereby, the accuracy and reliability of the impedance values can be further increased and the accuracy of the obtained hematocrit values can thus be further enhanced.

In yet another embodiment of the present invention, a blood temperature of the patient is sensed and the at least one hematocrit value is adjusted with respect to the sensed blood temperature. It have been found that the resistivity of whole blood increases with an increase in hematocrit and a decrease in temperature and this finding can thus be used to further increase the accuracy of the obtained hematocrit values.

In yet another embodiment, an activity level of the patient is sensed at predetermined intervals of time, the activity level values corresponding to the sensed activity levels of the patient are stored; and the development of the sensed activity level is monitored by comparing successive activity level values. It can be checked whether a predetermined number of successive sensed activity level values are below a predetermined reference activity level together with a check whether a predetermined number of successive hematocrit values are below a predetermined reference hematocrit level and if the predetermined number of successive sensed activity level values are below the predetermined reference activity level and the predetermined number of successive hematocrit values are below the predetermined reference hematocrit level, it can be determined that the monitored condition is deteriorating.

According to further embodiments, a breath rate of the patient is sensed at predetermined intervals of time; a minute respiratory volume of the patient is sensed at the predetermined intervals of time; the breath rate values corresponding to sensed breath rates and minute respiratory volume values corresponding to sensed minute respiratory volumes are stored; and the developments of the breath rate and the minute respiratory volume are monitored. This can be used to check whether a predetermined number of successive breath rate values are above a predetermined breath rate level and whether a predetermined number of successive minute respiratory volume values is below a predetermined minute respiratory volume, which, in turn, can be used to determine that shallow breathing occur if the predetermined number of successive breath rate values are above the predetermined breath rate level and the predetermined number of successive minute respiratory volume values are below the predetermined minute respiratory volume. If shallow breathing is determined and the predetermined number of successive hematocrit values are below the predetermined hematocrit level, the condition can be determined to be deteriorating.

In one embodiment of the present invention, it is checked whether the present hematocrit level is within a predetermined hematocrit level range and a delivery of diuretics is adjusted such that the hematocrit level is maintained within the predetermined hematocrit level range. Alternatively, it is checked whether present hematocrit level is within a predetermined hematocrit level range and if the present hematocrit level is found to be outside the predetermined range, a presence of a disorder of a kidney function of the patient is determined.

In yet another alternative, it is checked whether the present hematocrit level is within a predetermined hematocrit level range and if the present hematocrit level is found to be outside the predetermined range, it is indicated that blood values of the patient are abnormal.

In a further embodiment of the present invention, the medical lead is provided with distancing means, for example, distance elements cuffs attached to the lead. In another example, a basket arrangement is arranged at the lead such that the electrodes are surrounded. The cuffs, distance elements, or basket arrangement are preferably made in a material that not affects the impedance adversely. Thereby, if the electrodes of the medical lead are to be placed inside a blood vessel, it is possible to secure that the electrodes of the lead is held in the bloodstream and do not come into contact with the vessel wall, which may lead to overgrowth of the electrodes. This may,in turn, lead to impaired results due to degraded contact with the blood and/or increased current drawn due to the fact that a higher current may be needed to measure the impedance.

According to still another embodiment, the medical lead is J-shaped and the electrode surfaces are placed on the inside of the curvature of the lead. Thereby, if the electrodes are placed in inside a cavity of the heart, it may be ensured that the electrodes do not come into contact with a wall of the cavity, which may lead to overgrowth of the electrodes. This may,in turn, lead to impaired results due to degraded contact with the blood and/or increased current drawn due to the fact that a higher current may be needed to measure the impedance.

In yet another embodiment of the present invention, the medical lead comprising electrodes is provided with cuffs. The cuffs are preferably made in a material that not affects the impedance adversely. Thereby, if the electrodes are placed in inside a cavity of the heart, it may be ensured that the electrodes do not come into contact with a wall of the cavity, which may lead to overgrowth of the electrodes. This may,in turn, lead to impaired results due to degraded contact with the blood and/or increased current drawn due to the fact that a higher current may be needed to measure the impedance.

Furthermore, in another embodiment, the electrodes are located at a portion of the lead where overgrowth is unlikely, for example, just above or below the tricuspid valve. The distance between the tip of the lead, which is attached in the apex, and the electrodes is preferably about 6 to 10 cm or, more preferably, about 4 to 8 cm. Thereby, it may be ensured that a proper blood contact is achieved and undesired electrode tissue overgrowth or in-growth is avoided.

As will be apparent to those skilled in the art, the methods of the present invention, as well as preferred embodiments thereof, are suitable to realize as a computer program or a computer readable medium.

The features that characterize the invention, both as to organization and to method of operation, together with further objects and advantages thereof, will be better understood from the following description used in conjunction with the accompanying drawings. It is to be expressly understood that the drawings is for the purpose of illustration and description and is not intended as a definition of the limits of the invention. These and other objects attained, and advantages offered, by the present invention will become more fully apparent as the description that now follows is read in conjunction with the accompanying drawings.

BRIEF DESCRIPTION OF THE DRAWINGS

In the following detailed description, reference will be made to the accompanying drawings, of which:

Fig. 1 is schematic diagram showing a medical device implanted in a patient in which device the present invention can be implemented.

Fig. 2 is block diagram of the primary functional components of an embodiment of the implantable medical device according to the present invention.

Fig. 3 is block diagram of the primary functional components of another embodiment of the implantable medical device according to the present invention.

Fig. 4 is a flow chart illustrating the steps in accordance with one embodiment of the method of the present invention.

Fig. 5 is a diagram showing the linear relationship between resistance and hematocrit level for different frequencies.

Fig. 6 is block diagram of an embodiment of an impedance circuit for measuring complex impedance of blood of a patient in accordance with the present invention.

Fig. 7 is a schematic diagram showing an embodiment of the electrode arrangements and medical lead according to the present invention.

Fig. 8a is a schematic diagram showing another embodiment of the electrode arrangements and medical lead according to the present invention.

Fig. 8b is a schematic diagram showing a further embodiment of the electrode arrangements and medical lead according to the present invention.

DETAILED DESCRIPTION OF THE INVENTION

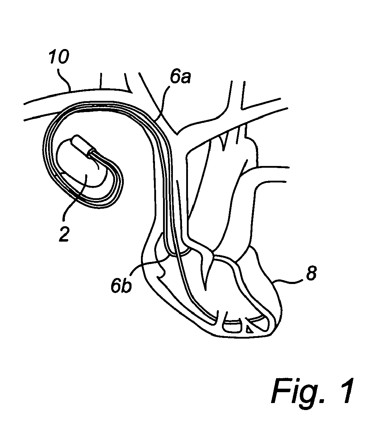

With reference to Fig. 1 there is shown a schematic diagram of a medical device implanted in a patient in which device the present invention can be implemented. As seen, this embodiment of the present invention is shown in the context of a pacemaker 2 implanted in a patient (not shown). The pacemaker 2 comprises a housing being hermetically sealed and biological inert. Normally, the housing is conductive and may, thus, serve as an electrode. One or more pacemaker leads, where only two are shown in Fig. 1 namely a ventricular lead 6a and an atrial lead 6b, are electrically coupled to the pacemaker 2 in a conventional manner. The leads 6a, 6b extend into the heart 8 via a vein 10 of the patient. One or more conductive electrodes for receiving electrical cardiac signals and/or for delivering electrical pacing to the heart 8 are arranged near the distal ends of the leads 6a, 6b. As the skilled man in the art realizes, the leads 6a, 6b may be implanted with its distal end located in either the atrium or ventricle of the heart 8.

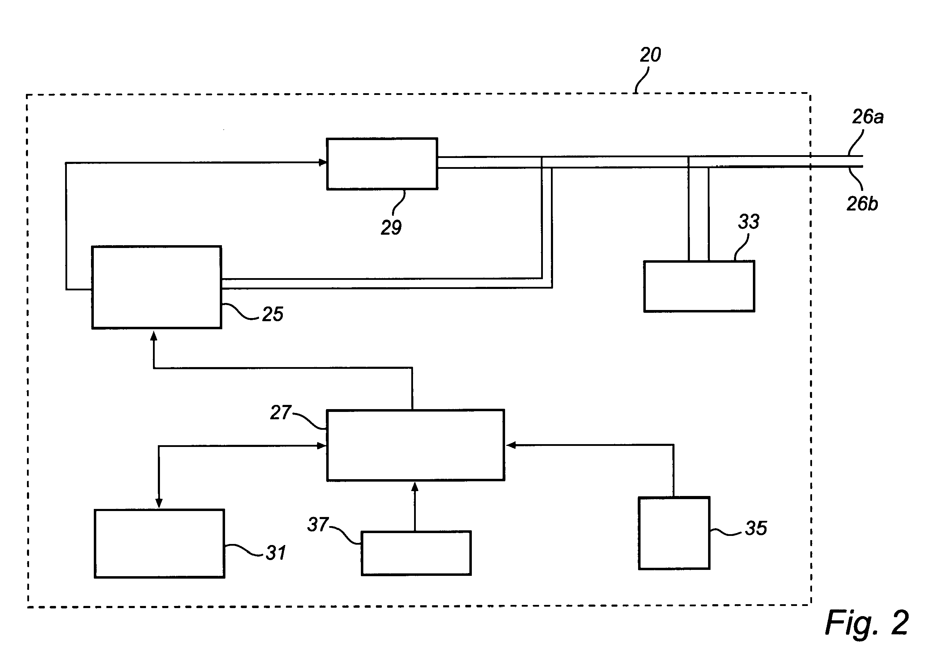

With reference now to Fig. 2, the configuration including the primary components of an embodiment of the present invention will be described. The illustrated embodiment comprises an implantable medical device 20, such as the pacemaker shown in Fig. 1, and leads 26a and 26b, of the same type as the leads 6a and 6b shown in Fig. 1, for delivering signals between the implantable medical device 20. The leads 26a, 26b may be unipolar or bipolar, and may include any of the passive or active fixation means known in the art for fixation of the lead to the cardiac tissue. As an example, the lead distal tip (not shown) may include a tined tip or a fixation helix. The leads 26a, 26b comprises one or more electrodes (as described with reference to fig. 1), such a tip electrode or a ring electrode, arranged to, inter alia, transmit pacing pulses for causing depolarization of cardiac tissue adjacent to the electrode(-s) generated by a pace pulse generator 25 under influence of a control circuit 27 comprising a microprocessor. The control circuit 27 controls, inter alia, pace pulse parameters such as output voltage and pulse duration.

Moreover, an impedance circuit 29 is adapted to carry out impedance measurements of the blood of the patient. The impedance circuit 29 is arranged to apply excitation current pulses between a first electrode and a second electrode arranged to positioned, for example, within a heart of the patient. The first and second electrode may also be positioned outside the heart. The impedance circuit 29 is also arranged to measure the voltage between a third and fourth electrode arranged, for example, at a lead 26a, or 26b. The third and fourth electrode are arranged such that they can be located within the heart of the patient, for example, in a vein/artery of the heart. In one embodiment, the impedance measurements are biventricular impedance measurements between the tip and the ring of an atrial lead or a ventricular lead. According to another embodiment, tri-polar measurements are used to perform the impedance measurements where the current is sent out between an RV-tip (i.e. the distal electrode in a bipolar lead located in right ventricle) and an RV-coil (i.e. the conductor in a bipolar lead having a helical configuration located in the right ventricle) and the voltage is measured between an RV-ring (i.e. the proximal electrode in a bipolar lead located in right ventricle) and the RV-coil. In yet another embodiment, a lead with 2 to 4 electrodes is placed free-flowing in a vein/artery.

Further, the impedance circuit 29 is coupled to the control circuit 27, where processing of the obtained impedance signals can be performed. A memory circuit 31 is connected to the control circuit 27, which memory circuit 31 may include a random access memory (RAM) and/or a non-volatile memory such as a read-only memory (ROM). Detected signals from the patients heart are processed in an input circuit 33 and are forwarded to the microprocessor of the control circuit 27 for use in logic timing determination in known manner. Furthermore, the implantable medical device 20 according to the present invention may comprise a position detecting sensor 35 arranged to detect, for example, a predetermined, specific body position of the patient. The position detecting sensor 35 is connected to the controller 27. The implantable medical device 20 is powered by a battery 37, which supplies electrical power to all electrical active components of the medical device 20. Data contained in the memory circuit 31 can be transferred to a programmer (not shown) via a programmer interface (not shown) for use in analyzing system conditions, patient information, etc.

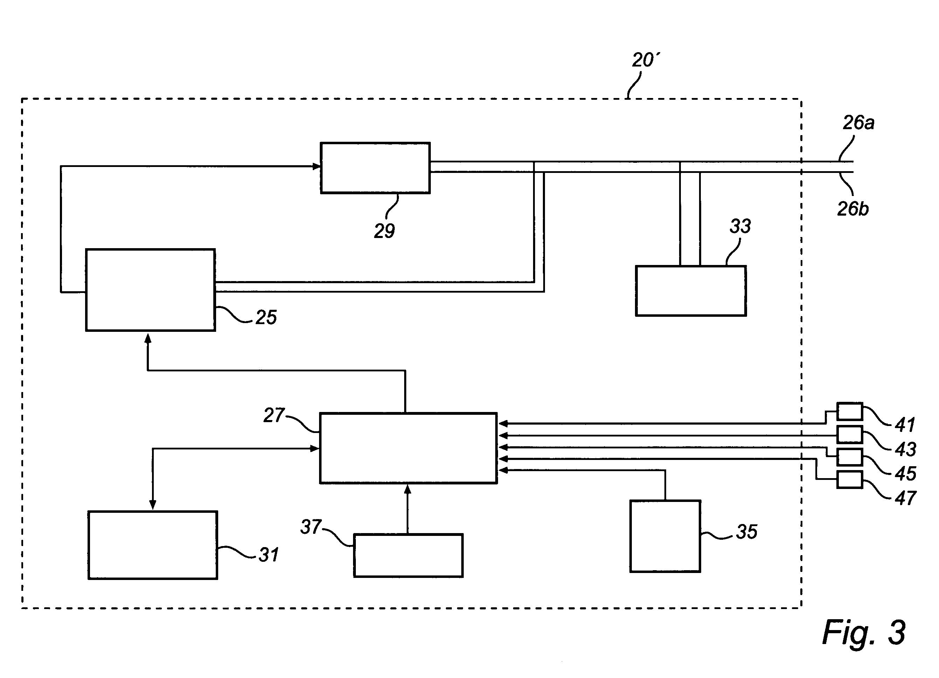

With reference to Fig. 3, another embodiment of the present invention will be described. In Fig. 2 and 3 similar parts are denoted with the same reference numerals. According to this embodiment, the implantable medical device 20' may include a heart rate sensor 41 connected to the controller 27 and a breathing rate sensor 43 connected to the controller 27. The controller 27 is adapted to, by sending a triggering signal, instruct the impedance circuit 29 to synchronize at least one impedance measurement session with a specific heartbeat in the respiration cycle of the patient and to calculate the at least one impedance value as a mean value of the measured impedance signal during the specific heartbeat during a predetermined number of respiration cycles. According to an alternative embodiment of the present invention the controller 27 is adapted to, by sending a triggering signal, instruct impedance circuit 29 to synchronize the impedance measurement session with a part of a specific heartbeat in the respiration cycle of the patient; and to calculate the at least one impedance value as a mean value of the measured impedance signal during the part of the heartbeat during a predetermined number of respiration cycles.

Furthermore, the medical device 20' according to the present invention may include a blood temperature sensor 45 connected to the controller 27. The controller 27 may be adapted to adjust a hematocrit value with respect to a sensed blood temperature in accordance with conventional manner as discussed, for example, in "The changes in blood resistivity with hematocrit and temperature" by Mohapatra SN and Hill DW, Eur. J. Intensive CARE MED., 1975, Dec: 1(4): 153-62.

In addition, the implantable medical device 20' may include an activity level sensor 47, such as an accelerometer, adapted to sense an activity level of the patient, the activity level sensor 47 being connected to the controller 27.

The controller 27 may be adapted to instruct the activity level sensing means 45 to sense the activity level at predetermined intervals of time. The sensed activity level values corresponding to the sensed activity levels of the patient can be stored in the memory 31. The controller 27 may be adapted to monitor the development of the sensed activity level by comparing successive activity level values. In an embodiment, this activity level trend is used to check whether a predetermined number of successive sensed activity level values are below a predetermined reference activity level. A check whether a predetermined number of successive hematocrit values are below a predetermined reference hematocrit level is also performed. If the predetermined number of successive sensed activity level values are below the predetermined reference activity level and the predetermined number of successive hematocrit values are below the predetermined reference hematocrit level, it is determined that a condition, for example, CHF is deteriorating. This can also be communicated to a programmer (not shown) via a programmer interface (not shown). The patient can be provided with an indication of the occurrence of this condition. For example, the medical device may include an alarm means adapted to cause the device to vibrate or to deliver a beeping sound in order to alert the patient of the situation. In an embodiment, the alarm means is integrated into the controller.

Moreover, the implantable medical device 20' may also include a sensor for sensing a minute respiratory volume of the patient 47 connected to the controller 27. This can be performed at predetermined intervals of time synchronized with sensing sessions for sensing a breath rate of the patient using breathing rate sensor 43. Values for minute respiratory volume corresponding to sensed breath rates and sensed minute respiratory volumes, respectively, can be stored in the memory 31. The controller 27 may be adapted to monitor the development of the breath rate and the minute respiratory volume by using successive values corresponding to sensed breath rates and sensed minute respiratory volumes, respectively, stored in the memory 31 to check whether a predetermined number of successive breath rate values are above a predetermined breath rate level and whether a predetermined number of successive minute respiratory volume values is below a predetermined minute respiratory volume. If the predetermined number of successive breath rate values are above the predetermined breath rate level and the predetermined number of successive minute respiratory volume values are below the predetermined minute respiratory volume, shallow breathing can be determined. If shallow breathing is determined and the predetermined number of successive hematocrit values are below the predetermined hematocrit level, it can be determined that the monitored condition, for example, CHF is deteriorating. This can also be communicated to a programmer (not shown) via a programmer interface (not shown). The patient can also be provided with an indication of the occurrence of this condition. For example, the medical device may include an alarm means adapted to cause the device to vibrate or to deliver a beeping sound in order to alert the patient of the situation. In an embodiment, the alarm means is integrated into the controller.

In another embodiment of the present invention, the controller 27 is adapted to check whether a present hematocrit level is within a predetermined hematocrit level range and, by sending a triggering signal, instruct a means for delivering diuretics to the patient (not shown) to adjust a delivery of diuretics such that the hematocrit level is maintained within the predetermined hematocrit level range. The patient can also be provided with an indication of the occurrence of this condition. For example, the medical device may include an alarm means adapted to cause the device to vibrate or to deliver a beeping sound in order to alert the patient of the situation. In an embodiment, the alarm means is integrated into the controller.

According to embodiments of the present invention, the controller is adapted to check whether a present hematocrit level is within a predetermined hematocrit level range and, if the present hematocrit level is found to be outside the predetermined range, a presence of a disorder of a kidney function of the patient can be determined. A notification of the occurrence of this conditions can be sent to a programmer (not shown) via a programmer interface (not shown) or the patient can be provided with an indication of the occurrence of this condition. For example, the medical device may include an alarm means adapted to cause the device to vibrate or to deliver a beeping sound in order to alert the patient of the situation. In an embodiment, the alarm means is integrated into the controller.

As will be appreciated by those of ordinary skille in the art, only one, some of or all of the following features: the heart rate sensor 41, the breathing rate sensor 43, the blood temperature sensor 45, the sensor for sensing a minute respiratory volume of the patient 47, the position detector 35, or the means for delivering diuretics to the patient may be included in the medical device according to the present invention.

In yet another embodiment, the controller is adapted to check whether a present hematocrit level is within a predetermined hematocrit level range and, if the present hematocrit level is found to be outside the predetermined range, it can be determined that blood values of the patient are abnormal. This can be transferred to a programmer (not shown) via a programmer interface (not shown) or the patient can be provided with an indication of the occurrence of this condition. For example, the medical device may include an alarm means adapted to cause the device to vibrate or to deliver a beeping sound in order to alert the patient of the situation. In an embodiment, the alarm means is integrated into the controller.

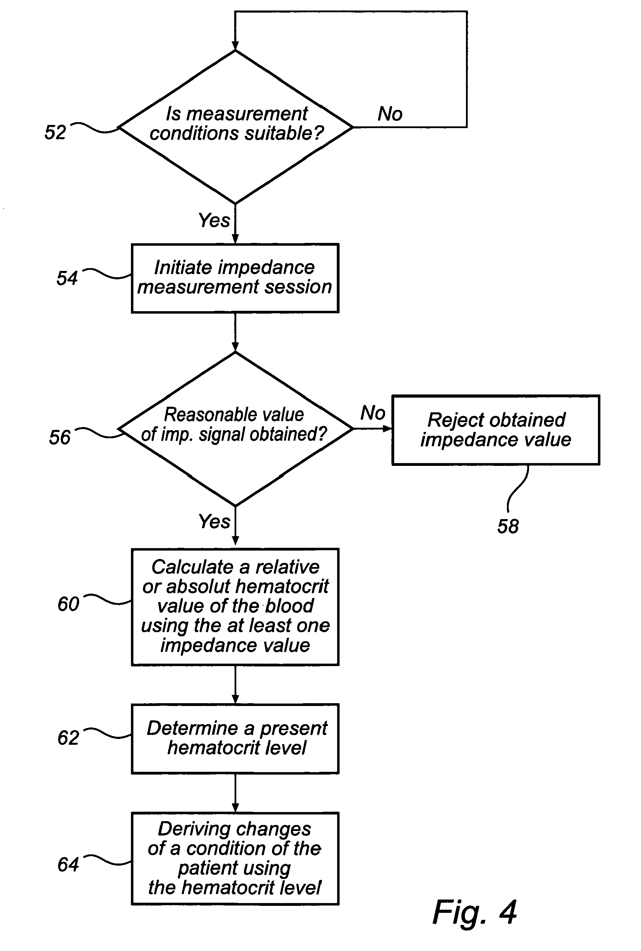

Referring now to Fig. 4, a high-level description of the method according to the present invention will be given.

The controller may optionally perform a check whether the measurements conditions are suitable before the controller 27 sends a triggering signal to the impedance circuit 29, which is in an idle mode. As will be described below, the measurements conditions can be specified to obtain more accurate blood impedance values. For example, a condition for initiating the impedance sensing session may be that a sensed activity level of the patient is within a predetermined range. The activity level can be sensed be means of an activity sensor incorporated in the medical device in accordance with conventional practice within the art. That is, the impedance sensing session is initiated only if the activity level signal is within the predetermined range. In case of this measurement condition check, a measurement condition obtaining procedure step is executed before the actual check is performed. Thus, optionally, a measurement condition obtaining procedure step and measurement condition check 52 may be performed before the controller 27, at step 54, sends a triggering signal to the impedance circuit 29, which sets the impedance circuit 29 in an active mode where the sensing circuit 29 initiates an impedance sensing session.

There are a number of possible impedance configurations, i.e. ways of injecting current between two electrodes in the pacemaker and then to measure the voltage the current provokes between the electrodes. For example, impedance configurations can be uni-polar, bi-polar, tri-polar or quadro-polar. The configuration denominated as bi-polar means, in practice, a configuration where the current and the voltage is sent out and measured between the same two electrodes. When one of the electrodes used in a bi-polar measurement is the housing or the case, the configuration is called uni-polar. For example, in Fig. 1, between the housing of the pacemaker 2 and a right ventricular electrode arranged at the distal end of lead 6a. A tri-polar configuration uses three electrodes, i.e. the current injection and the voltage measurement share one electrode. In quadro-polar measurements, the current is sent out between electrodes and the voltage is measured between two entirely different electrodes, i.e. in this case there are four electrodes involved. As mentioned above, in one embodiment of the present invention, the impedance measurements are biventricular impedance measurements between the tip and the ring of an atrial lead or a ventricular lead. According to another embodiment, tri-polar measurements are used to perform the impedance measurements where the current is sent out between an RV-tip (i.e. the distal electrode in a bipolar lead located in right ventricle) and an RV-coil (i.e. the conductor in a bipolar lead having a helical configuration located in the right ventricle) and the voltage is measured between an RV-ring (i.e. the proximal electrode in a bipolar lead located in right ventricle) and the RV-coil. In yet another embodiment, a lead with 2 to 4 electrodes is placed free flowing in a vein/artery.

The impedance circuit performs at least one impedance sensing session to obtain at least one blood impedance value corresponding to an impedance of whole blood of the patient and the at least one obtained impedance value is communicated to the controller 27. The controller 27 may optionally, perform a validity check in order to check or judge whether the obtained impedance value is reasonable or valid. This can be performed, for example, by checking that the obtained value is within a preset range including the preceding value. If the obtained value is found to be invalid, i.e. the value is outside the preset range, the value or signal is rejected. In one embodiment, a new impedance measurement session is initiated after a delay period of a predetermined length and if this is repeated a preset number of times without a valid signal have been obtained, the impedance measurement circuit returns to the idle mode. Accordingly, a validity check may be executed as an option at step 56 before the controller 27, at step 60, calculates at least one relative or absolute value of an amount of hematocrit in the blood of the patient using the at least one impedance value. This calculation can be performed in accordance with conventional practice. For example, it has been shown that the resistivity of the blood is highly dependent upon the hematocrit (Hct), where Hematocrit denotes the percent volume of the red blood cells in the whole blood volume. This dependence has an exponential nature as can be seen in the equation given below, relating the Hct value to the resistivity of the blood:

$$r = 0.537 e^{0.025\,Hct} \qquad \text{(eqt. A).}$$

According to another approach, the relation between resistivity and Hct can be expressed as:

$$\rho = 0.586 \frac{1 + 0.0125\,Hct}{1 - 0.01\,Hct} \qquad \text{(eqt. B)}$$

, where $\rho$ = resistivity of blood [$\Omega$m] and Hct = hematocrit [%].
The hematocrit values most often range between 36.1 - 44.3 % for women and 40.7 - 50 % for men. Within these specified intervals, the two equations [eqt. A] and [eqt. B] provide almost the same result, which implies that both may be used when calculating a hematocrit value from a measured blood resistivity (impedance).

Returning now to Fig. 4, if the value is outside the preset range, the value or signal is rejected at step 58. At step 62, the controller 27 determines a present hematocrit level by means of the at least one hematocrit value. Finally, at step 64, a change of a condition can be derived using the at least one hematocrit value. It should however be noted that, as the skilled man easily realizes, the above-mentioned steps are not necessarily performed in the order discussed above.

The present hematocrit level as well as the at least one hematocrit value and impedance value can be stored in the memory 31 in consecutive time order. The present hematocrit level together with preceding levels can be used to derive a change of a condition of the patient. For example, conditions such as CHF, cancer, chronic kidney disease, diabetes, rheumatoid arthritis, inflammatory bowel disease and HIV / AIDS can be derived. Moreover, the present hematocrit level can be communicated or transferred to an external device, for example, a programmer (not shown) via a programmer interface (not shown), for example, a telemetry device. Hence, at step 54, a patient condition check procedure may be performed to derive a change of a condition of the patient. For example, the present hematocrit values may be compared with stored hematocrit level values obtained in earlier sessions to monitor, for example, changes and/or trends of the development of the impedance. Thereby, it can be derived whether a condition of the patient influencing the blood impedance and, thus, the hematocrit level is changing, for example, congestive heart failure.

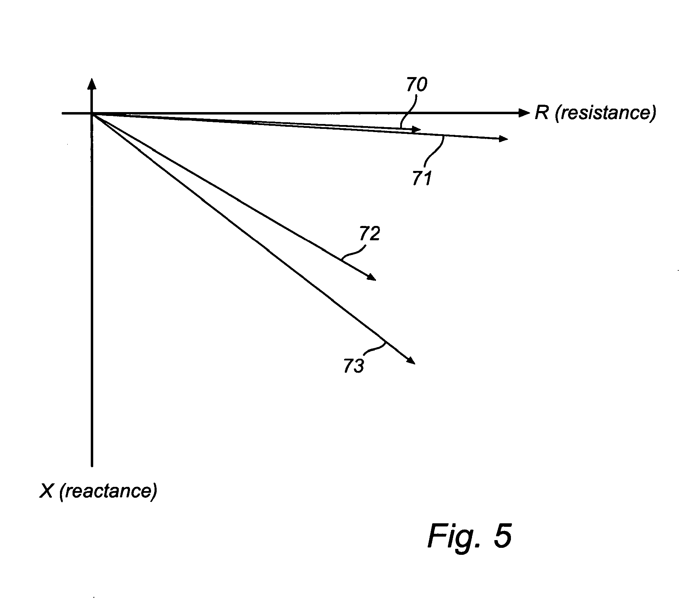

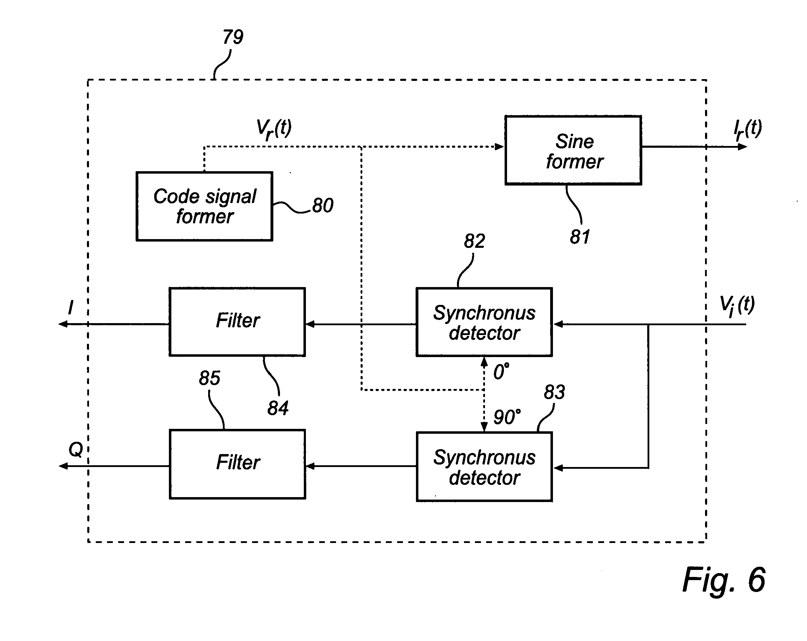

With reference now to Fig. 5 and 6, measurements of complex impedance of the blood of the patient will be discussed. As discussed above, both the blood cell plasma and the intracellular liquid are mainly resistive at frequencies 1 Hz - 500 kHz. The blood cell membranes are electrically considered as capacitors. At low frequencies the AC current pass around the cells, but at high frequencies the current also pass through the cells. At low frequencies there will be a low phase shift and at high frequencies the phase angel ($\alpha$) will be around -35 degrees. A high hematocrit level will give a higher phase angle than at a lower hematocrit level. This can be used to calculate a hematocrit value, for example, by using the findings in "The impedance measurement of human blood in relations to the hemorheological determinants", Deng LH et al., International Journal of Bioelectromagnetism, Vol. 4, No. 2, 2002, pp. 167-168. Deng et al. demonstrate a linear relationship between resistance and hematocrit see Fig. 5, where the line 70 indicates a low hematocrit value and a low frequency, line 71 indicates a high hematocrit value and low frequency, line 72 indicates a low hematocrit value and high frequency, and line 73 indicates a high hematocrit value and a high frequency. It can be seen that at a low hematocrit value (line 70 and 71), the resistance R is lower at low frequencies but independent at high frequencies (line 72 and 73). At high frequencies the reactance X is higher for higher hematocrit values (see line 73) compared to low hematocrit values (see line 72). Thus, the phase angle ($\alpha$) becomes negative at higher frequencies.

Referring now to Fig. 6, an embodiment of an impedance circuit adapted for measuring complex impedance of the blood of the patient will be described. The impedance circuit 79 comprises a code signal former circuit 80 adapted to control the impedance measurements by synchronizing the different circuits of the impedance circuit 79. Alternatively, the code signal former circuit 80 may be arranged in the controller 27. Moreover, the impedance circuit 79 includes a sine former circuit 81 driven by the code signal former circuit 80, which sine former circuit 81 is adapted to deliver a stepwise sine current:

$$I_r = A_r \cdot \sin \omega t \qquad (1)$$

to the test object, i.e. the blood of the patient. The current is applied between a first electrode and a second electrode, which electrodes are adapted to be located, for example, within a heart of the patient. The resulting voltage difference at the input of a first synchronous detector 82 and at a second synchronous detector 83 connected to a respective electrode is:

$$V_i(t) = A_i \cdot \sin(\omega_i t + \varphi) \qquad (2)$$

The first synchronous detector 82 and the second synchronous detector 83 are adapted to perform simultaneous in-phase and quadrature signal processing. The detectors 82 and 83 function as analog multipliers with mutually quadrature stepwise-approximated harmonic waveforms. The code signal former circuit 80 delivers reference signals to the first synchronous detector 82 and the second synchronous detector 83, respectively, in accordance with:

$$V_r(t) = A_r \cdot \sin \omega_r t \qquad (3)$$

for the synchronous (0°) signal and

$$V_r(t) = A_r \cdot \cos \omega_r t \qquad (4)$$

for the quadrature signal.

In first synchronous detector 82, equation (2) and (3) are multiplied, and the output is:

$$V_i(t) \cdot V_r(t) = \frac{1}{2} A_i A_r \cdot \cos \varphi - \frac{1}{2} A_i A_r \cdot \cos(2\omega t + \varphi) \quad (5)$$

In the second synchronous detector 83, equation (2) and (3) are multiplied, and the output is:

$$V_i(t) \cdot V_r(t) = \frac{1}{2} A_i A_r \cdot \sin \varphi + \frac{1}{2} A_i A_r \cdot \sin(2\omega t + \varphi) \quad (6)$$

Both DC and AC components of the multiplications (5) and (6) contain the impedance information but only the first component is used in this embodiment. The second component (double frequency pulsation) is filtered out by low-pass filtering in a first low-pass filer 84 and a second low-pass filter 85 connected to the first synchronous detector 82 and the second synchronous detector 83, respectively. The resulting signals at the output is:

$$V_{OI}(t) = V_i(t) \cdot \overline{V_r}(t) = \frac{A_i A_r}{2} \cos \varphi \qquad (7)$$

for the synchronous component and:

$$V_{OQ}(t) = V_i(t) \cdot \overline{V_r}(t) = \frac{A_i A_r}{2} \sin \varphi \qquad (8)$$

for quadrature component.

Equation (7) and (8) are the real and imaginary values for the impedance vector and hence reflects the resistive component and the reactance component, respectively, of the impedance. The impedance values can be utilized by the controller 27 to calculate relative or absolute values of the hematocrit in the blood of the patient using, for example, the relationship discussed with reference to Fig. 5.

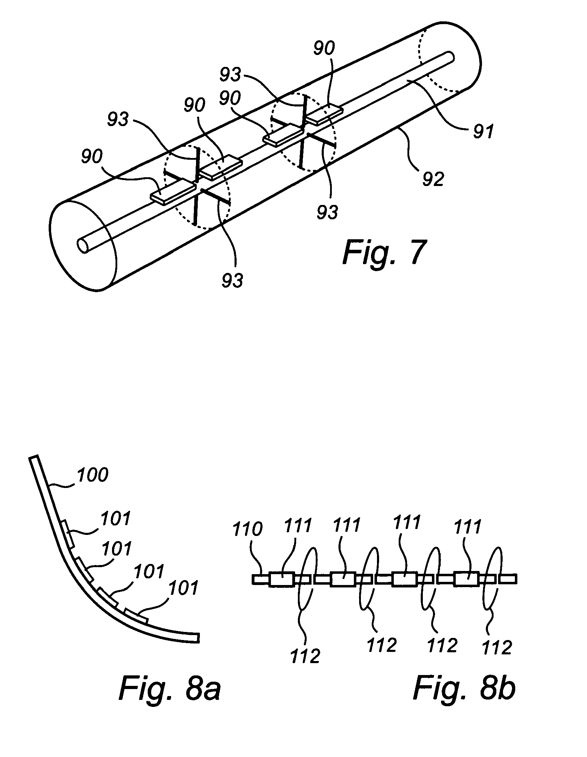

With reference now to Fig. 7, a further embodiment of the present invention will be discussed. In this embodiment, electrodes 90 of a medical lead 91 is placed in a blood vessel 92 of the heart, which blood vessel is schematically shown in Fig. 7. The medical lead 91 is provided with distancing means 93, for example, cuffs or distance elements attached to the lead. The distance elements 93 are preferably made in a material that not affects the impedance adversely. Thereby, it is possible to secure that the electrodes of the lead is held in the bloodstream and do not come into contact with the vessel wall, which may lead to overgrowth or in-growth of the electrodes. This may, in turn, lead to impaired results due to degraded contact with the blood and/or increased current drawn due to the fact that a higher current may be needed to measure the impedance.

Turning now to Fig. 8a, another embodiment of the present invention will be discussed. In this embodiment, the medical lead 100 is J-shaped and the electrode surfaces 101 are placed on the inside of the curvature of the lead 100. Thereby, it is possible to ensure that electrodes that are to be placed inside a cavity of the heart do not come into contact with a wall of the cavity, which may lead to overgrowth or in-growth of the electrodes. This may,in turn, lead to impaired results due to degraded contact with the blood and/or increased current drawn due to the fact that a higher current may be needed to measure the impedance.

Referring to Fig. 8b, another embodiment of the present invention will be discussed. The medical lead 110 comprising electrodes 111 is provided with cuffs 112. The cuffs 112 are preferably made in a material that not affects the impedance adversely. Thereby, if the electrodes are placed in inside a cavity of the heart, it may be ensured that the electrodes do not come into contact with a wall of the cavity, which may lead to overgrowth of the electrodes. This may,in turn, lead to impaired results due to degraded contact with the blood and/or increased current drawn due to the fact that a higher current may be needed to measure the impedance.

Furthermore, in another embodiment, the electrodes are located at a portion of the lead where overgrowth is unlikely, for example, just above or below the tricuspid valve. The distance between the tip of the lead, which is attached in the apex, and the electrodes is preferably about 6 to 10 cm or, more preferably, about 4 to 8 cm. Thereby, it may be ensured that a proper blood contact is achieved and that undesired electrode tissue overgrowth or in-growth is avoided.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

**Claims**

1. A method for detecting a change of a condition of a patient using an implantable medical device (10; 20; 20') being connectable to said patient in at least one electrode configuration arranged such that impedance measurement sessions can be performed within the body of said patient, **characterized by** the steps of:

   initiating (54) at least one impedance measurement session using said electrode configuration to obtain at least one impedance value corresponding to an impedance of whole blood of said patient, wherein said electrode configuration comprises at least two electrodes arranged such that they can be located within a heart of said patient;
   calculating (60) at least one relative or absolute value of an amount of hematocrit in the blood of said patient using said at least one impedance value; and
   determining (62, 64) a present hematocrit level by means of said at least one hematocrit value, wherein a change of said condition can be derived from said present hematocrit level of said patient.

2. The method according to claim 1, further comprising the steps of:

   repeating (56) said at least one impedance measurement session at predetermined intervals of time, wherein a sequence of hematocrit values over time can be obtained; and
   storing (56) said hematocrit values.

3. The method according to claim 2, wherein the step of deriving (64) comprises the step of:

   monitoring the development of said hematocrit level by comparing successive hematocrit values.

4. The method according to claim 1-3, wherein initiation (54) of said at least one impedance measurement session is correlated with the cardiac cycle of said patient such that said session is initiated before the onset of systole.

5. The method according to claim 1-4, wherein said condition is Congestive Heart Failure (CHF).

6. The method according to any one of preceding claims, wherein the step of initiating (54) comprises the step of:

initiating a first impedance measurement session using a current at a first frequency using said electrode configuration in order to obtain at least one complex impedance value corresponding to an impedance of the blood of said patient at said first frequency; and

initiating a second impedance measurement session using a current at a second frequency using said electrode configuration in order to obtain at least one complex impedance value corresponding to an impedance of the blood of said patient at said second frequency, wherein said second frequency is higher than said first frequency.

7. The method according to claim 6, wherein the step of calculating comprises the steps of:

determining a phase shift between said first complex impedance value obtained in said first impedance measurement session and said second complex impedance value obtained in said second impedance measurement session; and

calculating said at least one value of the amount of hematocrit in the blood of said patient using said phase shift.

8. The method according to any one of preceding claims, further comprising the step of:

calculating said at least one impedance value as a mean value of the measured impedance signal over a predetermined period of time.

9. The method according to claim 1-7, further comprising the steps of:

sensing a heart rate of the patient;
sensing a breathing rate of the patient;
synchronizing said at least one impedance measurement session with a specific heartbeat in the respiration cycle of said patient; and
calculating said at least one impedance value as a mean value of the measured impedance signal during said heartbeat.

10. The method according to claim 9, further comprising the step of:

calculating said at least one impedance value as a mean value for said heartbeat over a predetermined number of respiration cycles of said patient.

11. The method according to any one of preceding claims 1-7, further comprising the step of:

sensing a heart rate of the patient;
sensing a breathing rate of the patient;
synchronizing said impedance measurement session with a part of a specific heartbeat in the respiration cycle of said patient; and
calculating said at least one impedance value as a mean value of the measured impedance signal during said part of said heartbeat.

12. The method according to any one of preceding claims, further comprising the steps of:

sensing a blood temperature of said patient; and
adjusting said at least one hematocrit value with respect to said sensed blood temperature.

13. The method according to claim 3, further comprising the steps of
sensing an activity level of said patient at predetermined intervals of time;
storing activity level values corresponding to said sensed activity levels of said patient; and
monitoring the development of said sensed activity level by comparing successive activity level values.

14. The method according to claim 13, further comprising the steps of
checking whether a predetermined number of successive hematocrit values are below a predetermined reference hematocrit level;
checking whether a predetermined number of successive sensed activity level values are below a predetermined reference activity level; and
if said predetermined number of successive sensed activity level values are below said predetermined reference activity level and said predetermined number of successive hematocrit values are below said predetermined reference hematocrit level, determining that said condition is deteriorating.

15. The method according to claim 3, further comprising the steps of:

sensing a breath rate of said patient at predetermined intervals of time;
sensing a minute respiratory volume of said patient at said predetermined intervals of time;
storing breath rate values corresponding to sensed breath rates and minute respiratory volume values corresponding to sensed minute respiratory volumes; and monitoring the development of said breath rate and said minute respiratory volume.

16. The method according to claim 15, further comprising the steps of:

checking whether a predetermined number of successive breath rate values are above a predetermined breath rate level;
checking whether a predetermined number of successive minute respiratory volume values is below a predetermined minute respiratory volume;
determining shallow breathing if said predetermined number of successive breath rate values are above said predetermined breath rate level and said predetermined number of successive minute respiratory volume values are below said predetermined minute respiratory volume; and
if shallow breathing is determined and said predetermined number of successive hematocrit values are below said predetermined hematocrit level, determining that said condition is deteriorating.

17. The method according to any one of preceding claims, further comprising the steps of:

checking whether said present hematocrit level is within a predetermined hematocrit level range; and
adjusting a delivery of diuretics such that said hematocrit level is maintained within said predetermined hematocrit level range.

18. The method according to claim 1, further comprising the steps of:

checking whether said present hematocrit level is within a predetermined hematocrit level range; and
if said present hematocrit level is found to be outside said predetermined range, determining presence of a disorder of a kidney function of said patient.

19. The method according to claim 1, further comprising the steps of:

checking whether said present hematocrit level is within a predetermined hematocrit level range; and
if said present hematocrit level is found to be outside said predetermined range, indicating that blood values of said patient are abnormal.

20. An implantable medical device for detecting a change of a condition of a patient, said medical device (10; 20; 20') being connectable to said patient in at least one electrode configuration arranged such that impedance measurement sessions can be performed within the body of said patient, **characterized by**
an impedance circuit (29) adapted to measure at least one impedance of the blood of said patient at said electrode configuration, said electrode configuration comprising at least two electrodes arranged such that they can be located within a heart of said patient, wherein said impedance circuit (29) is adapted to, upon receiving a triggering signal, initiate at least one impedance sensing session to obtain at least one impedance value corresponding to an impedance of whole blood of said patient; and
a controller (27) adapted to calculate at least one relative or absolute value of an amount of hematocrit in the blood of said patient using said at least one impedance value; and to determine a present hematocrit level by means of said at least one hematocrit value, wherein a change of said condition of said patient can be derived from a present hematocrit level.

21. The implantable medical device according to claim 20, further comprising a memory (31), and wherein said controller (27) is adapted to, by sending a triggering signal, instruct said impedance circuit (29) to repeat said at least one impedance measurement session at predetermined intervals of time, wherein a sequence of hematocrit values over time can be obtained; and wherein said memory (31) is adapted to store said hematocrit values.

**22.** The implantable medical device according to claim 21, wherein the controller (27) is adapted to monitor the development of said hematocrit level by comparing successive hematocrit values.

**23.** The implantable medical device according to claim 20-22, wherein the controller (27) is adapted to , by sending a triggering signal, instruct said impedance circuit (29) to initiate of said at least one impedance measurement session in correlation with the cardiac cycle of said patient such that said session is initiated before the onset of systole.

**24.** The implantable medical device according to claim 20-23, wherein said condition is Congestive Heart Failure (CHF).

**25.** The implantable medical device according to any one of preceding claims 20-24,
wherein said controller (27) is adapted to:

, by sending a triggering signal, instruct said impedance circuit (29) to initiate a first impedance measurement session using a current at a first frequency using said electrode configuration to obtain at least one complex impedance value corresponding to an impedance of the blood of said patient at said first frequency; and
, by sending a triggering signal, instruct said impedance circuit (29) to initiate a second impedance measurement session using a current at a second frequency using said electrode configuration to obtain at least one complex impedance value corresponding to an impedance of the blood of said patient at said second frequency, wherein said second frequency is higher than said first frequency.

**26.** The implantable medical device according to claim 25, wherein said controller (27) is adapted to:

determine a phase shift between said first complex impedance value obtained in said first impedance measurement session and said second complex impedance value obtained in said second impedance measurement session; and
calculate said at least one value of the amount of hematocrit in the blood of said patient using said phase shift.

**27.** The implantable medical device according to any one of preceding claims 20-26, wherein said controller (27) is adapted to calculate said at least one impedance value as a mean value of the measured impedance signal over a predetermined period of time.

**28.** The implantable medical device according to claim 20-27, further comprising:

means for sensing a heart rate (41) of the patient connected to said controller (27);
means for sensing a breathing rate (43) of the patient connected to said controller (27);

wherein said controller (27) is adapted to:

, by sending a triggering signal, instruct said impedance circuit (29) to synchronize said at least one impedance measurement session with a specific heartbeat in the respiration cycle of said patient; and
calculate said at least one impedance value as a mean value of the measured impedance signal during said heartbeat.

**29.** The implantable medical device according to claim 28, wherein said controller (27) is adapted to calculate said at least one impedance value as a mean value for said heartbeat over a predetermined number of respiration cycles of said patient.

**30.** The implantable medical device according to any one of preceding claims 20-27, further comprising:

means for sensing a heart rate (41) of the patient connected to said controller (27);
means for sensing a breathing rate (43) of the patient connected to said controller (27);

wherein said controller (27) is adapted to:

, by sending a triggering signal, instruct said impedance circuit (29) to synchronize said impedance measurement session with a part of a specific heartbeat in the respiration cycle of said patient; and
calculate said at least one impedance value as a mean value of the measured impedance signal during said part of said heartbeat.

**31.** The implantable medical device according to any one of preceding claims 20-30, further comprising:

means for sensing a blood temperature (45) of said patient; and

wherein said controller (27) is adapted to adjust said at least one hematocrit value with respect to said sensed blood temperature.

**32.** The implantable medical device according to claim 20-31, further comprising:

means for sensing an activity level (47) of said patient at predetermined intervals of time; and

wherein said memory (31) is adapted to store activity level values corresponding to said sensed activity levels of said patient; and
wherein said controller (27) is adapted to monitor the development of said sensed activity level by comparing successive activity level values.

**33.** The implantable medical device according to claim 32, wherein said controller (27) is adapted to:

check whether a predetermined number of successive hematocrit values are below a predetermined reference hematocrit level;
check whether a predetermined number of successive sensed activity level values are below a predetermined reference activity level; and
if said predetermined number of successive sensed activity level values are below said predetermined reference activity level and said predetermined number of successive hematocrit values are below said predetermined reference hematocrit level, determine that said condition is deteriorating.

**34.** The implantable medical device according to any one of preceding claims 20-33, further comprising:

means for sensing a breath rate (41) of said patient at predetermined intervals of time;
means for sensing a minute respiratory volume (47) of said patient at said predetermined intervals of time; and wherein
said memory (31) is adapted to store breath rate values corresponding to sensed breath rates and minute respiratory volume values corresponding to sensed minute respiratory volumes; and

wherein said controller (27) is adapted to monitor the development of said breath rate and said minute respiratory volume.

**35.** The implantable medical device according to claim 34, wherein said controller (27) is adapted to:

check whether a predetermined number of successive breath rate values are above a predetermined breath rate level;
check whether a predetermined number of successive minute respiratory volume values is below a predetermined minute respiratory volume;
determine shallow breathing if said predetermined number of successive breath rate values are above said predetermined breath rate level and said predetermined number of successive minute respiratory volume values are below said predetermined minute respiratory volume; and
if shallow breathing is determined and said predetermined number of successive hematocrit values are below said predetermined hematocrit level, determine that said condition is deteriorating.

**36.** The implantable medical device according to any one of preceding claims 20-35, further comprising means for delivering diuretics to said patient, wherein said controller (27) is adapted to
check whether said present hematocrit level is within a predetermined hematocrit level range; and
, by sending a triggering signal, instruct said means for delivering diuretics to said patient to adjust a delivery of diuretics such that said hematocrit level is maintained within said predetermined hematocrit level range.

**37.** The implantable medical device according to any one of preceding claims 20-36,
wherein said controller (27) is adapted to:

check whether said present hematocrit level is within a predetermined hematocrit level range; and
if said present hematocrit level is found to be outside said predetermined range, determine presence of a disorder of a kidney function of said patient.

38. The implantable medical device according to any one of preceding claims 20-37, wherein said controller (27) is adapted to
check whether said present hematocrit level is within a predetermined hematocrit level range; and
if said present hematocrit level is found to be outside said predetermined range, indicate that blood values of said patient are abnormal.

39. Computer readable medium comprising instructions for bringing a computer to perform a method according to any one of the preceding claims 1-19.

40. A computer program product, directly loadable into a memory (31) of an implantable medical device (10; 20; 20'), comprising software code portions for causing said medical device (10; 20; 20') to perform steps in accordance with claims 1-19.

Fig. 1

Fig. 2

Fig. 3

EP 1 825 807 A2

52 — Is measurement conditions suitable?  → No

Yes

54 — Initiate impedance measurement session

56 — Reasonable value of imp. signal obtained?  → No → Reject obtained impedance value

58

Yes

60 — Calculate a relative or absolut hematocrit value of the blood using the at least one impedance value

62 — Determine a present hematocrit level

64 — Deriving changes of a condition of the patient using the hematocrit level

Fig. 4

70

71

72

73

R (resistance)

X (reactance)

*Fig. 5*

Fig. 6

EP 1 825 807 A2

Fig. 7

Fig. 8a

Fig. 8b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9909883 A

- US 5526808 A

### Non-patent literature cited in the description

- **ANDRONE et al.** Hemodilution is Common in Patients with Advanced Heart Failure. *Circulation,* 2003, vol. 107, 226-229
- **SILVERBERG et al.** The Cardio Renal Anemia (CRA) Syndrome: Congestive Heart Failure, Cronic Kidney Insufficiency, and Anemia. *Dialysis Times, News & Views from RPI,* vol. 10 (1
- **CHA K.** An electronic method for rapid measurement of hematocrit in blood samples. *Physiol. Meas.,* 1994, vol. 15 (5), 129-137
- **GHEORGHE A. POP et al.** Catheter-based impedance measurements in the right atrium for continuously monitoring hematocrit and estimating blood viscosity changes; an in vivo feasibility study in swine. *Biosensors and Bioelectronics,* 2004, vol. 19, 1685-1693

- **CHA K.** An electronic method for rapid measurement of hematocrit in blood samples. *Physiol. Meas.,* 1994, vol. 15, 129-137
- *Circulation,* 21 January 2003, vol. 107 (2), 226-9
- **MOHAPATRA SN ; HILL DW.** The changes in blood resistivity with hematocrit and temperature. *Eur. J. Intensive CARE MED.,* vol. 1 (4), 153-62
- **DENG LH et al.** The impedance measurement of human blood in relations to the hemorheological determinants. *International Journal of Bioelectromagnetism,* 2002, vol. 4 (2), 167-168